# EUROPÄISCHE PATENTSCHRIFT

(11) **EP 2 313 079 B1**
(45) Veröffentlichungstag und Bekanntmachung des Hinweises auf die Patenterteilung: **12.10.2016**
(21) Anmeldenummer: 09780875.2
(22) Anmeldetag: 21.07.2009
(51) Int. Cl.: A61K 8/81, A61Q 5/06, A61K 8/86

(54) **MITTEL FÜR KERATINHALTIGE FASERN, ENTHALTEND MINDESTENS EIN SPEZIELLES AMPHIPHILES KATIONISCHES POLYMER UND MINDESTENS EIN SPEZIELLES AMPHIPHILES, ANIONISCHES POLYMER**
PRODUCT FOR KERATIN-CONTAINING FIBRES COMPRISING AT LEAST ONE SPECIFIC AMPHIPHILIC CATIONIC POLYMER, AND AT LEAST ONE SPECIFIC AMPHIPHILIC ANIONIC POLYMER
PRÉPARATION POUR FIBRES KÉRATINIQUES, CONTENANT AU MOINS UN POLYMÈRE CATIONIQUE AMPHIPHILE SPÉCIAL ET AU MOINS UN POLYMÈRE ANIONIQUE AMPHIPHILE SPÉCIAL

(30) Priorität: 18.08.2008 DE 102008038105
(43) Veröffentlichungstag der Anmeldung: 27.04.2011
(73) Patentinhaber: Henkel AG & Co. KGaA, 40589 Düsseldorf (DE)
(72) Erfinder: KNAPPE, Thorsten, 22869 Schenefeld (DE); NOLL, Marcus, 22850 Norderstedt (DE)
(86) Internationale Anmeldenummer: PCT/EP2009/059355
(87) Internationale Veröffentlichungsnummer: WO 2010/020503

(56) Entgegenhaltungen:
- DE-A1-102007 008 089
- US-A1- 2006 013 785
- US-A1- 2006 134 049
- US-A1- 2008 119 413
- US-B1- 6 852 815
- ISP: "AquaStyle 300" INTERNET CITATION 6. Dezember 2006 (2006-12-06), Seiten 1-94, XP007910767 Gefunden im Internet: URL:http://www.ispjapan.co.jp/pc_refguide/ pdf/AquaStyle_300_jp.pdf> [gefunden am 2009-12-03]
- RAYMOND RIGOLETTO ET AL: "Polyquaternium-69: A New Fixative Polymer with Enhanced Styling Benefits" INTERNET CITATION 1. Januar 2007 (2007-01-01), XP007910768 Gefunden im Internet: URL:http://www.cosmeticsciencetechnology.c om/articles/samples/1281.pdf> [gefunden am 2009-12-03]

## Beschreibung

Die vorliegende Erfindung betrifft Mittel zur Haarbehandlung, enthaltend eine Kombination mindestens eines speziellen amphiphilen, kationischen Polymers mit mindestens einem speziellen amphiphilen, anionischen Polymer, die Verwendung dieser Mittel zur temporären Verformung und/oder zur Pflege keratinhaltiger Fasern und Haargele auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Wenn ein Stylingmittel als Gel zur Anwendung kommt, hat es sich als Vorteilhaft erwiesen, diese als klare, transparente Gele bereitzustellen. Diese klaren Gele empfindet der Verbraucher als ästhetisch ansprechend, insbesondere dann, wenn zusätzlich Gasblasen eingearbeitet sind. Leider lassen sich in herkömmliche Gelformulierungen oft Gasblasen schwer einarbeiten. Gelang es, Gasblasen in die herkömmlichen Rezepturen einzuarbeiten, waren die Gasblasen nicht lagerstabil eingearbeitet und entwichen aus dem Gel.

Stylingmittel zu entwickeln, die alle gewünschten Eigenschaften in Kombination aufweisen, bereitet nach wie vor Schwierigkeiten. Insbesondere gilt dies für die Kombination von ästhetischen Gesichtpunkten einerseits und starkem und flexiblem Halt andererseits. Um einen starken Halt zu vermitteln muss das fixierend wirkende Polymer gut auf der keratinhaltigen Faser haften und einen hinreichend harten Film bilden. Dennoch der resultierende Polymerfilm dem Faserkollektiv keine Taktilität eines Bretts verleihen, sondern den Fasern einen Grad an Flexibilität ermöglichen, ohne dass die aufgeprägte Formgebung des Faserkollektivs, d.h. z.B. eine Frisur, verloren geht.

Aufgabe der vorliegenden Erfindung war es daher, ein Mittel zur temporären Verformung keratinischer Fasern zur Verfügung zu stellen, das sich durch einen hohen Haltegrad auszeichnet und keine Filmplaken bildet. Ferner soll das Mittel in Form eines klaren, transparenten Gels vorliegen, in das sich Gasblasen lagerstabil einarbeiten lassen.

Es wurde nunmehr überraschenderweise gefunden, dass dies durch die erfindungsgemäße Polymerkombination (*vide infra*) erreicht werden kann. Mit der erfindungsgemäßen Polymerkombination können transparente Gele hergestellt werden, in die sich Gasblasen dauerhaft einarbeiten lassen, wobei die resultierenden Gele eine hervorragende Frisurfestigung bewirken.

Ein erster Gegenstand der vorliegenden Erfindung sind daher Mittel zur Behandlung keratinhaltiger Fasern, insbesondere menschlichem Haar, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 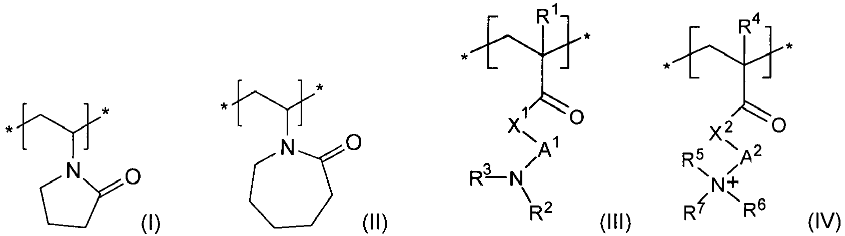 worin
   R¹ und R⁴ unabhängig voneinander stehen für ein Wasserstoffatom oder eine Methylgruppe, X¹ und X² unabhängig voneinander stehen für ein Sauerstoffatom oder eine Gruppe NH,
   A¹ und A² unabhängig voneinander stehen für eine Gruppe Ethan-1,2-diyl, Propan-1,3-diyl oder Butan-1,4-diyl,
   R², R³, R⁵ und R⁶ unabhängig voneinander stehen für eine (C₁ bis C₄)-Alkylgruppe,
   R⁷ steht für eine (C₈ bis C₃₀)-Alkylgruppe
      und
(b) mindestens ein vernetztes amphiphiles, anionisches Polymer, umfassend mindestens eine Struktureinheit der Formel (V) und mindestens eine Struktureinheit der Formel (VI), 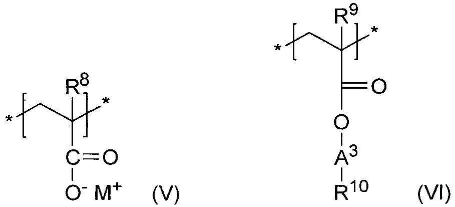 worin
   R⁸ und R⁹ stehen unabhängig voneinander für ein Wasserstoffatom oder eine Methylgruppe, R¹⁰ steht für eine (C₈ bis C₃₀)-Alkylgruppe,
   M⁺ steht für ein physiologisch verträgliches Kation und
   A³ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe *-(CH₂CH₂O)ₓ-(CH₂CHMeO)_{y}-* worin die Summe x + y für eine ganze Zahl von 5 bis 35 steht und x und y größer als Null sind.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl.

Beispiele für erfindungsgemäße (C₈ bis C₃₀)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Gemäß obiger Formeln und allen folgenden Formeln steht eine chemische Bindung, die mit dem Symbol * gekennzeichnet ist, für eine freie Valenz des entsprechenden Strukturfragments.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Insbesondere sind als physiologisch verträgliche Kationen zur Kompensation der negativen Ladung des amphiphilen, anionischen Polymers (b) Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom geeignet. Letztere werden beispielsweise durch Protonierung primärer, sekundärer oder tertiärer organischer Amine mit einer Säure, oder durch permanente Quaternisierung besagter organischer Amine gebildet. Beispiele dieser kationischen organischen Ammoniumverbindungen sind 2-Ammonioethanol und 2-Trimethylammonioethanol.

Besonders vorteilhaft erweisen sich die Eigenschaften des erfindungsgemäßen Mittels, wenn es cremeförmig, bevorzugt gelförmig vorliegt, insbesondere in Form eines klaren, transparenten Gels. Diese bevorzugte Form der Konfektionierung wird später im Detail beschrieben.

Nachfolgende amphiphile, kationische Polymere finden erfindungsgemäß bevorzugt in den erfindungsgemäßen Mitteln Einsatz, wenn die amphiphilen, kationischen Polymere eines oder mehrere der folgenden Merkmale erfüllen:
- R¹ und R⁴ bedeuten jeweils eine Methylgruppe,
- X¹ steht für eine Gruppe NH,
- X² steht für eine Gruppe NH,
- A¹ und A² stehen unabhängig voneinander für Ethan-1,2-diyl oder Propan-1,3-diyl,
- R², R³, R⁵ und R⁶ stehen unabhängig voneinander für Methyl oder Ethyl, (besonders bevorzugt für Methyl),
- R⁷ steht für eine (C₁₀ bis C₂₄)-Alkylgruppe, insbesondere für Decyl (Caprinyl), Undecyl, Dodecyl (Lauryl), Tridecyl, Tetradecyl (Myristyl), Pentadecyl, Hexadecyl (Cetyl), Octadecyl (Stearyl), Eicosyl (Arachyl) oder Docosyl (Behenyl).

Es ist erfindungsgemäß bevorzugt, die Struktureinheit der Formel (III) aus aus mindestens einer Struktureinheit der Formel (III-1) bis (III-8) auszuwählen

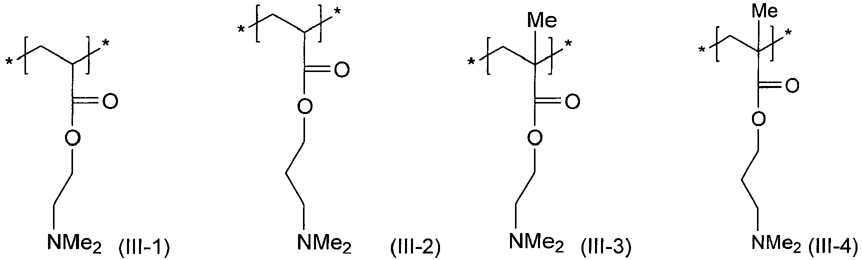

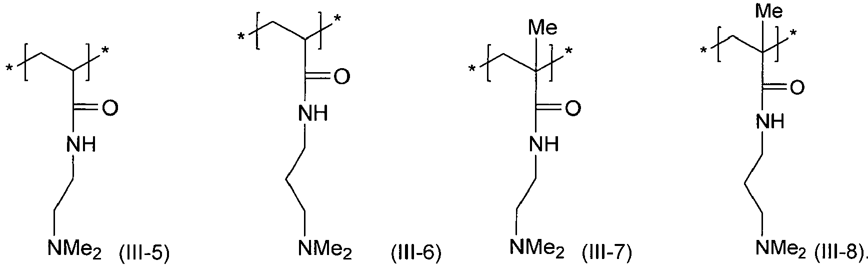

Außerdem erwies es sich als besonders bevorzugt, als Struktureinheit der Formel (III) die Struktureinheit gemäß Formel (III-7) und/oder der Formel (III-8) zu wählen. Die Struktureinheit der Formel (III-8) ist erfindungsgemäß eine ganz besonders bevorzugte Struktureinheit.

Ferner stellte es sich mit Blick auf die Lösung der Aufgabe als bevorzugt heraus, wenn die Struktureinheit der Formel (IV) ausgewählt wird, aus mindestens einer Struktureinheit der Formeln (IV-1) bis (IV-8) 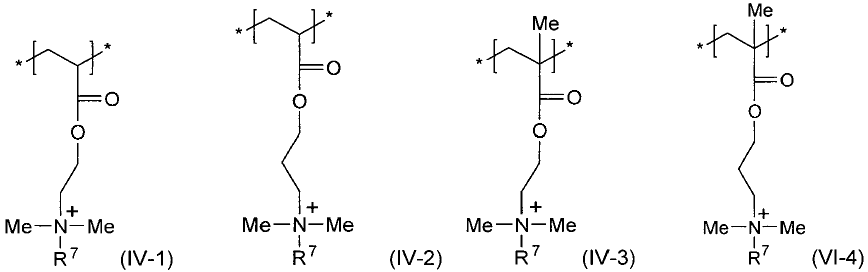 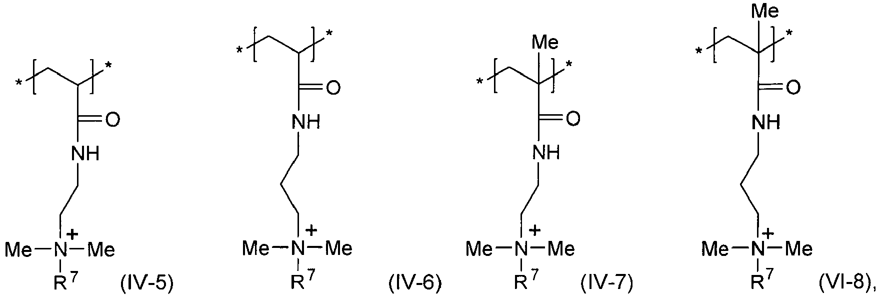 worin R⁷ jeweils für eine (C₈ bis C₃₀)-Alkylgruppe steht.

Als wiederum besonders bevorzugte Struktureinheit der Formel (IV) gelten die Struktureinheiten der Formel (IV-7) und/oder der Formel (IV-8), worin jeweils R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl). Die Struktureinheit der Formel (IV-8) stellt erfindungsgemäß eine ganz besonders bevorzugte Struktureinheit der Formel (IV) dar.

Ein ganz besonders bevorzugtes amphiphiles, kationisches Polymer umfasst mindestens eine Struktureinheit der Formel (I), mindestens eine Struktureinheit der Formel (II), mindestens eine Struktureinheit der Formel (III-8) und mindestens eine Struktureinheit der Formel (IV-8), 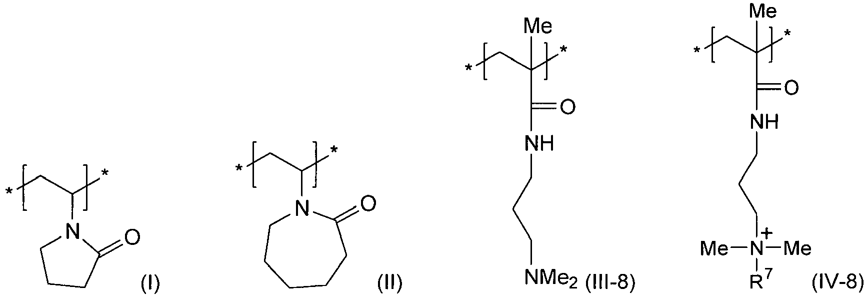 worin R⁷ steht für eine (C₈ bis C₃₀)-Alkylgruppe (insbesondere für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl)).

Die erfindungsgemäßen amphiphilen, kationischen Polymere weisen bevorzugt ein Molekulargewicht von 10000 g/mol bis 50000000 g/mol, insbesondere von 50000 g/mol bis 5000000 g/mol, besonders bevorzugt von 75000 g/mol bis 1000000 g/mol.

Ein ganz besonders bevorzugtes erfindungsgemäßes amphiphiles, kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle® 300 (28-32 Gew.-% Aktivsubstanz in Ethanol-Wasser-Gemisch) von der Firma ISP vertrieben wird.

Im Sinne der Erfindung bevorzugte Mittel enthalten die oben beschriebenen amphiphilen, kationischen Polymere in einer Menge von 0,05 Gew.-% bis 15,0 Gew.-%, besonders bevorzugt von 0,05 Gew.-% bis 10,0 Gew.-%, ganz besonders bevorzugt von 0,1 bis 5,0 Gew.%, jeweils bezogen auf das Gewicht des Mittels.

Weiterhin enthält das erfindungsgemäße Mittel zusätzlich zu den zuvor definierten amphiphilen, kationischen Polymeren mindestens ein zuvor definiertes amphiphiles, anionisches Polymer auf.

Diese amphiphilen, anionischen Polymere sind vernetzt. Unter "vernetzt" bzw. "Vernetzung" ist im Sinne der Erfindung die Verknüpfung von Polymerketten miteinander durch kovalente chemische Bindung unter Bildung eines Netzwerkes zu verstehen. Diese kovalente Verknüpfung der Polymerketten darf mittels direkter kovalenter Bindung erfolgen oder durch ein die Polymerketten verbrückendes Molekülfragment vermittelt werden. Das Molekülfragment bindet an die durch das Molekülfragment verbrückten Polymerketten jeweils mittels kovalenter chemischer Bindung. Unter "unvernetzt" ist im Sinne der Erfindung zu verstehen, dass keine zuvor definierte "Vernetzung" vorliegt. Die Vernetzung der vernetzen Ausführungsform der amphiphilen, anionischen Polymere (b) kann bevorzugt durch Verwendung mindestens eines vernetzenden Monomers bewerkstelligt werden. Dabei ist es wiederum bevorzugt die vernetzenden Monomere aus mindestens einer Verbindung der Gruppe zu wählen, die gebildet wird aus polyungesättigten aromatischen Monomeren (wie beispielsweise Divinylbenzol, Divinylnaphthalin, Trivinylbenzol), polyungesättigten alicyclischen Monomeren (wie beispielsweise 1,2,4-Trivinylcyclohexan), difunktionellen Estern der Phthalsäure (wie beispielsweise Diallylphthalat), polyungesättigte aliphatische Monomere (wie beispielsweise Diene, Triene,Tetraene wie Isopren, 1,3-Butadien, 1,5-Hexadien, 1,5,9-Decatrien, 1,9-Decadien, 1,5-Heptadien), Polyalkenylether (wie beispielsweise Triallylpentaerythritol, Diallylpentaerythritol, Diallylsucrose, Octaallylsucrose, Trimethylolpropandiallylether), polyungesättigte Ester von Polyalkoholen oder Polysäuren (wie beispielsweise 1,6-Hexandioldi(meth)acrylat, Tetramethylentri(meth)acrylat, Allylacrylat, Diallylitaconat, Diallylfumarat, Diallylmaleat, Trimethylolpropantri(meth)acrylat, Trimethylolpropandi(meth)acrylat, Polyethyleneglycoldi(meth)acrylat), Alkylenebisacrylamide (wie beispielsweise Methylenbisacrylamid, Propylenbisacrylamid) Hydroxy- und Carboxyderivate des Methylenbisacrylamids (wie beispielsweise N,N'-Bismethylolmethylenbisacrylamid), Polyethyleneglycoldi(meth)acrylate (wie beispielsweise Ethyleneglycoldi(meth)acrylat, Diethyleneglycoldi(meth)acrylat, Triethyleneglycoldi(meth)acrylat), polyungesättigte Silane (wie beispielsweise Dimethyldivinylsilan, Methyltrivinylsilan, Allyldimethylvinylsilan, Diallyldimethylsilan, Tetravinylsilan), N-Methylolacrylamid; N-alkoxy(meth)acrylamid, wobei die Alkoxygruppe eine (C₁ bis C₁₈)-Alkoxygruppe ist, ungesättigte hydrolysierbare Silane (wie beispielsweise Triethoxyvinylsilan, Trisisopropoxyvinylsilan, 3-Triethoxysilylpropylmethacrylat), hydrolyierbare Silane (wie beispielsweise Ethyltriethoxysilan, Ethyltrimethoxysilan), Epoxy-substituierte hydrolysierbare Silane (wie beispielsweise 2-(3,4-Epoxycyclohexyl)ethyltriethoxysilan, 3-Glycidoxypropyltrimethyoxysilan) Polyisocyanate (wie beispielsweise 1,4-Diisocyanatobutan, 1,6-Diisocyanatohexan, 1,4-Phenylenediisocyanat, 4,4'-Oxybis(phenylisocyanat), ungesättigte Epoxide (wie beispielsweise Glycidylmethacrylate, Allylglycidylether), Polyepoxide (wie beispielsweise Diglycidylether, 1,2,5,6-Diepoxyhexan, Ethylenglycoldiglycidylether), ethoxylierte Polyole (wie beispielsweise Diole, Triole und Diphenole, jeweils ethoxyliert mit 2 bis 100 mol Ethyleneoxid pro Mol Hydroxylgruppen und terminiert mit einer polymerisierbaren ungesättigten Gruppe, wie beispielsweise Vinylether, Allylether, Acrylateester, Methacrylateester; Beispiele umfassen Bisphenol A ethoxyliertes di(meth)acrylat, Bisphenol F ethoxyliertes Di(meth)acrylat, ethoxyliertes Trimethylolpropantri(meth)acrylate, Acrylat- und Methacrylatester von Polyolen mit mindestens zwei Acrylatester- oder Methacrylatester-Funktionalitäten (wie beispielsweise Trimethylolpropantriacrylat (TMPTA), Trimethylolpropanethoxylated (15) triacrylat (TMPEO15TA), Trimethylolpropandimethacrylat, Triethyleneglycoldimethacrylat (TEGDMA), mit 30 Mol Ethylenoxid ethoxylliertes Bisphenol A-dimethacrylat (EOBDMA)).

Besonders bevorzugte erfindungsgemäße Mittel sind dadurch gekennzeichnet, dass die amphiphilen, anionischen Polymere (b) eine Molmasse von 100 bis 500 kDa, vorzugsweise von 150 bis 400 kDa, weiter bevorzugt von 200 bis 300 kDa und insbesondere von 225 bis 275 kDa, aufweisen.

Im Sinne der Erfindung bevorzugte Mittel enthalten die amphiphilen, anionischen Polymere (b) in einer Menge von 0,05 Gew.-% bis 10,0 Gew.-%, besonders bevorzugt von 0,05 Gew.-% bis 5,0 Gew.-%, ganz besonders bevorzugt von 0,1 bis 2,0 Gew.%, jeweils bezogen auf das Gewicht des Mittels.

Die Polymere (a) und die Polymere (b) werden erfindungsgemäß bevorzugt in einem Gewichtsverhältnis [Polymer (a) zu Polymer (b)] von 1 zu 10 bis 10 zu 1, insbesondere von 1 zu 5 bis 5 zu 1, ganz besonders bevorzugt von 1 zu 3.5 bis 3.5 zu 1, eingesetzt.

Es ist erfindungsgemäß bevorzugt, wenn die amphiphilen, anionischen Polymere (b) aus der Gruppe der Copolymere b1 ausgewählt werden, die mindestens eine Struktureinheit der Formel (V-1), mindestens eine Struktureinheit der Formel (V-2) sowie mindestens eine Struktureinheit der Formel (VI) umfassen 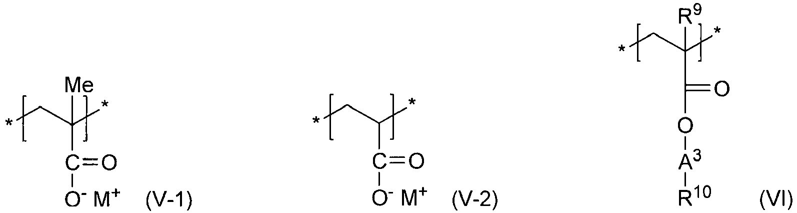 worin
M⁺ unabhängig voneinander für ein physiologisch verträgliches Kation steht,
R⁹ steht für ein Wasserstoffatom oder eine Methylgruppe (bevorzugt für eine Methylgruppe),
R¹⁰ steht für eine (C₈ bis C₃₀)-Alkylgruppe (insbesondere für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl)),
A³ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe *-(CH₂CH₂O)ₓ-(CH₂CHMeO)_{y}-* worin die Summe x + y für eine ganze Zahl von 5 bis 35 steht und x und y größer als Null sind (bevorzugt für eine Gruppe *-(CH₂CH₂O)ₓ-* worin x für eine ganze Zahl von 5 bis 30 steht).

Erfindungsgemäß bevorzugt verwendbare Copolymere b1 umfassen mindestens eine Struktureinheit der Formel (V-1), mindestens eine Struktureinheit der Formel (V-2) sowie mindestens eine Struktureinheit der Formel (VI-1) 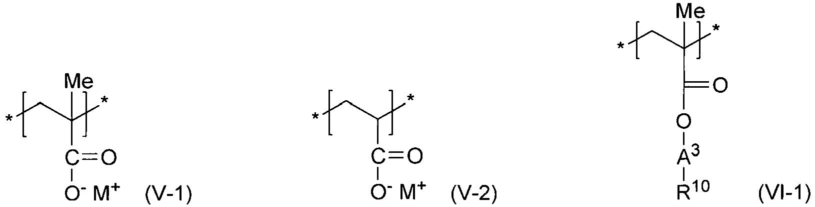 worin
M⁺ unabhängig voneinander für ein physiologisch verträgliches Kation steht,
R¹⁰ steht für eine (C₈ bis C₃₀)-Alkylgruppe (insbesondere für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl)) und
A³ steht für eine Gruppe *-(CH₂CH₂O)ₓ-* worin x für eine ganze Zahl von 5 bis 35, insbesondere von 15 bis 30 (15, 16, 17, 18, 19, 20, 21, 22, 23, 24, 25, 26, 27, 28, 29, 30), steht.

Zur Klarheit sei für einen Nichtfachmann erwähnt, dass gemäß Formel (VI) bzw. (VI-1) der Rest R¹⁰ stets an das Sauerstoffatom der Gruppe A³ bindet.

Wiederum bevorzugt sind solche amphiphilen, anionischen Polymere (b) einsetzbar, die aus Copolymeren b1 von Acrylsäure mit Methacrylsäure, mindestens einem (C₁ bis C₄)-Alkylacrylat und mindestens einem ethoxylierten Methacrylsäureester und/oder ethoxylierten Acrylsäureester ausgewählt werden.

Diese Copolymere b1 lassen sich durch die Formel (b1-1) 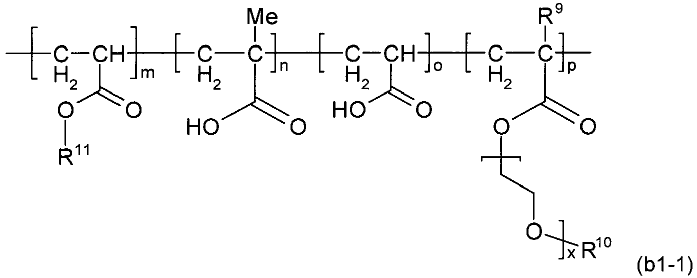 beschreiben, wobei
die Indices m, n, o und p je nach Molmasse des Polymers variieren,
R⁹ steht für ein Wasserstoffatom oder eine Methylgruppe,
R¹⁰ steht für einen Kohlenwasserstoffrest mit 8 bis 30, insbesondere mit 10 bis 24, Kohlenstoffatomen,
R¹¹ steht für eine (C₁ bis C₄)-Alkylgruppe (bevorzugt für -CH₃, -CH₂CH₃, -CHMe₂, -CH₂CH₂CH₃,-CH₂CHMeCH₃ oder -CH₂CH₂CH₂CH₃, ganz besonders bevorzugt für -CH₃ und/oder -CH₂CH₃),
x steht für 5 bis 35 (insbesondere für 15 bis 30).

Die Anordnung der Struktureinheiten in obiger Formel (b1-1) bedeutet nicht, daß es sich bei den Copolymeren b1 zwingend um Blockcopolymere handelt. Vielmehr können die Struktureinheiten im Molekül statistisch verteilt vorliegen.

Besonders bevorzugte erfindungsgemäße Mittel sind dadurch gekennzeichnet, daß sie als Copolymer b1 Copolymere von (C₁ bis C₄)-Alkylacrylat, Acrylsäure, Methacrylsäure und ethoxylierten (Meth)acrylsäureestern mit einer Molmasse von 100 bis 500 kDa, vorzugsweise von 150 bis 400 kDa, weiter bevorzugt von 200 bis 300 kDa und insbesondere von 225 bis 275 kDa, enthalten. Die Indizes m, n, o und p gemäß Ausführungsform der Formel (b1-1) sind entsprechend.

Die bevorzugten Mengenbereiche, in denen das bevorzugte Copolymer b1 in dem erfindungsgemäße Mittel bevorzugt eingesetzt wird, sowie die Mengen des amphiphilen, kationischen Polymers (a) und die Mengenverhältnisse zu dem amphiphilen, kationischen Polymer (a) entsprechen dem zuvor Gesagten (*vide supra*).

Besonders bevorzugte Copolymere b1 weisen 20 bis 30 EO-Einheiten auf (x = 20, 21, 22, 23, 24, 25, 26, 27, 28, 29, 30) und besitzen als Rest R einen Stearylrest oder Behenylrest.

Ein ganz besonders bevorzugtes Polymer (a) ist ein vernetztes, amphiphiles, anionisches Polymer, das unter die INCI-Bezeichnung Acrylates / Steareth-20 Methacrylate Crosspolymer fällt. Es besitzt 20 Einheiten Ethylenoxid (x gemäß Formel (II-a) = 20) und ist mit Stearylalkohol verethert (R³ gemäß Formel (II-a) = Stearyl) Solche Polymere werden beispielsweise mit dem Handelsnamen Aculyn^{®} 88 von der Firma Rohm & Haas in Form einer 28 bis 30 Gew.-%-igen Dispersion in Wasser vertrieben.

Ein besonders bevorzugtes Mittel, insbesondere in Form eines Gels, enthält in einem kosmetisch akzeptablen Träger
(a) mindestens ein amphiphiles, kationisches Polymer umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 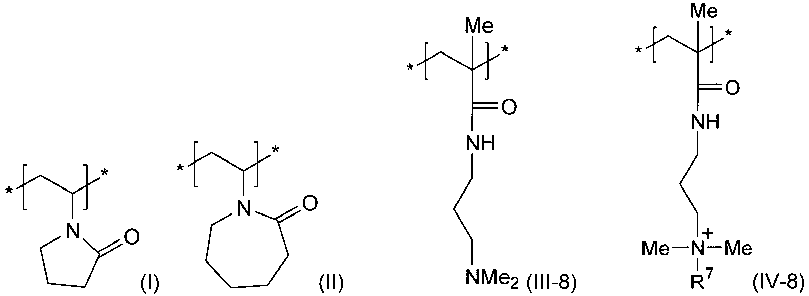 worin R⁷ steht für eine (C₈ bis C₃₀)-Alkylgruppe (insbesondere für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl)),
   und
(b) mindestens einem vernetzten amphiphilen, anionischen Polymer, umfassend mindestens eine Struktureinheit der Formel (V-1), mindestens eine Struktureinheit der Formel (V-2) sowie mindestens eine Struktureinheit der Formel (VI-1) 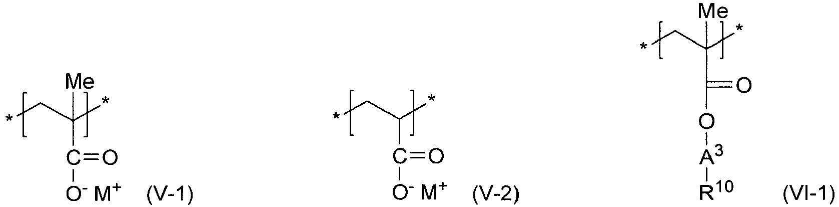 worin
   M⁺ unabhängig voneinander für ein physiologisch verträgliches Kation steht,
   R¹⁰ steht für eine (C₈ bis C₃₀)-Alkylgruppe (insbesondere für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl)) und
   A³ steht für eine Gruppe *-(CH₂CH₂O)ₓ-* worin x für eine ganze Zahl von 5 bis 35, insbesondere von 15 bis 30 (15, 16, 17, 18, 19, 20, 21, 22, 23, 24, 25, 26, 27, 28, 29, 30), steht.

Ein ganz besonders bevorzugtes Mittel, insbesondere in Form eines Gels, enthält in einem kosmetisch akzeptablen Träger
(a) mindestens ein amphiphiles, kationisches Polymer umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 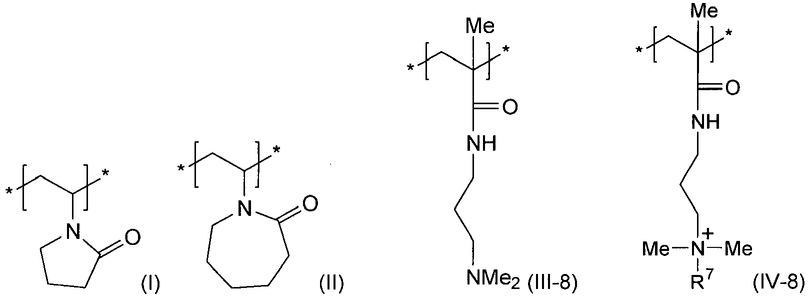 worin R⁷ steht für eine (C₈ bis C₃₀)-Alkylgruppe (insbesondere für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl)),
   und
(b) mindestens einem vernetzten amphiphilen, anionischen Polymer der Formel (b1-1) 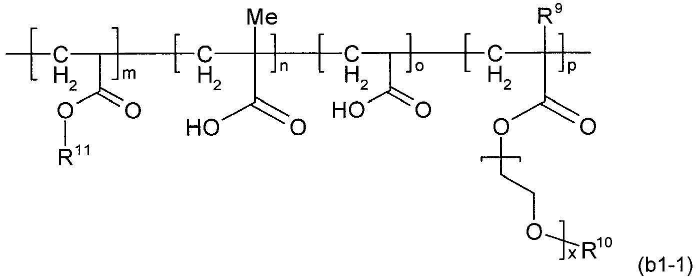 wobei
   die Indices m, n, o und p jeweils eine ganze Zahl größer Null bedeuten,
   R⁹ steht für ein Wasserstoffatom oder eine Methylgruppe,
   R¹⁰ steht für ein Kohlenwasserstoffrest mit einem bis 24 C-Atomen,
   R¹¹ steht für eine (C₁ bis C₄)-Alkylgruppe (bevorzugt für -CH₃, -CH₂CH₃, -CHMe₂, -CH₂CH₂CH₃, -CH₂CHMeCH₃ oder -CH₂CH₂CH₂CH₃, ganz besonders bevorzugt für -CH₃ und/oder -CH₂CH₃),
   x steht für 5 bis 35 (insbesondere für 15 bis 30).

Die Anordnung der Struktureinheiten in obiger Formel (b1-1) bedeutet nicht, daß es sich bei den Copolymeren b1 zwingend um Blockcopolymere handelt. Vielmehr können die Struktureinheiten im Molekül statistisch verteilt vorliegen.

Das erfindungsgemäße Mittel enthält in einer bevorzugten Ausführungsform zusätzlich zum amphiphilen, kationischen Polymer (a) und dem vernetzten amphiphilen, anionischen Polymer (b) weiterhin zusätzlich mindestens ein filmbildendes und/oder festigendes Polymer (c). Letzteres ist von den Polymeren (a) und (b) verschieden.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers wird häufig der so genannte curl-retention - Test angewendet.

Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch.

Das erfindungsgemäße Mittel enthält bevorzugt mindestens ein filmbildendes und/oder festigendes Polymer, das aus mindestens einem Polymer der Gruppe ausgewählt wird, die gebildet wird aus nichtionischen Polymeren, kationischen Polymeren, amphoteren Polymeren, zwitterionischen Polymeren und anionischen Polymeren.

Die zusätzlichen filmbildenden und/oder festigenden Polymere sind in dem erfindungsgemäßen Mittel bevorzugt in einer Menge von 0,01 Gew.-% bis 20 Gew.-%, insbesondere von 0,5 Gew.-% bis 15 Gew.-%, ganz besonders bevorzugt von 2,0 Gew.-% bis 10,0 Gew.-%, jeweils bezogen auf das Gewicht des Mittels, enthalten. Diese Mengenangaben gelten auch für alle in den erfindungsgemäßen Mitteln einsetzbaren nachfolgenden bevorzugten Typen der filmbildenden und/oder festigenden Polymere. Falls nachfolgend abweichende bevorzugte Mengen spezifiziert wurden, gelten letztere als wiederum noch bevorzugter Mengen.

Besonders bevorzugt eignen sich erfindungsgemäß solche Mittel, die neben den zuvor definierten amphiphilen, kationischen Polymeren zusätzlich mindestens ein filmbildendes und/oder festigende Polymer enthalten, welches aus mindestens einem Polymer der Gruppe ausgewählt wird, die gebildet wird aus
- nichtionischen Polymeren auf Basis ethylenisch ungesättigter Monomere, insbesondere aus
- Homopolymeren des N-Vinylpyrrolidons,
- nichtionischen Copolymeren des N-Vinylpyrrolidons,
- Homopolymeren und nichtionischen Copolymeren des N-Vinylcaprolactams,
- Copolymeren des (Meth)acrylamids,
- Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 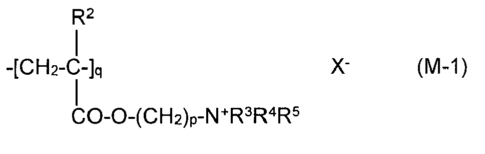 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X- ein physiologisch verträgliches organisches oder anorganisches Anion ist,
- anionischen Polymeren, welche Carboxylat- und/oder Sulfonatgruppen aufweisen,
- anionischen Polyurethanen.

Bevorzugt als zusätzliches filmbildendes und/oder festigendes Polymer geeignete nichtionische Polymere auf Basis ethylenisch ungesättigter Monomere, sind solche nichtionischen Polymere, welche mindestens eine der nachfolgenden Struktureinheiten enthalten 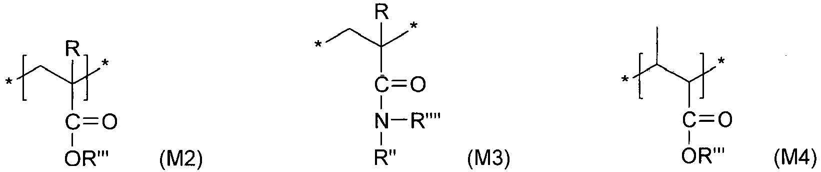 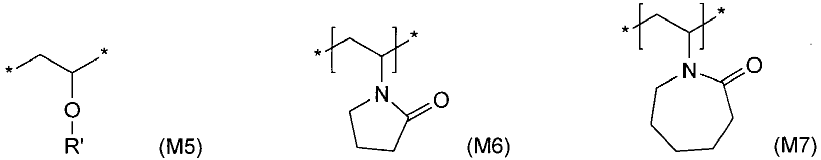 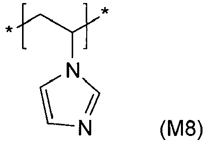 worin
- R: steht für ein Wasserstoffatom oder eine Methylgruppe,
- R': steht für ein Wasserstoffatom oder eine (C₁ bis C₄)-Acylgruppe,
- R" und R"": stehen unabhängig voneinander für eine (C₁ bis C₇)-Alkylgruppe oder ein Wasserstoffatom
- R"': steht für eine lineare oder verzweigte (C₁ bis C₄)-Alkylgruppe oder eine (C₂ bis C₄)-Hydroxyalkylgruppe.

Bevorzugte, nichtionische filmbildende und/oder nichtionische haarfestigende Polymere sind Homo-oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, wobei jeweils die Alkylgruppen dieser Monomere aus (C₁ bis C₃)-Alkylgruppen ausgewählt werden.

Für die erfindungsgemäßen Mittel besonders geeignete nichtionische Polymere auf Basis ethylenisch ungesättigter Monomere enthalten mindestens eine der nachfolgenden Struktureinheiten 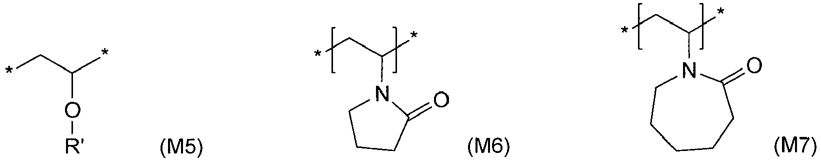 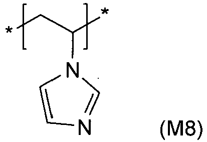 worin
- R': steht für ein Wasserstoffatom oder eine (C₁- bis C₃₀)-Acylgruppe, insbesondere für ein Wasserstoffatom oder eine Acetylgruppe.

Geeignet sind insbesondere Homopolymere des Vinylcaprolactams oder des Vinylpyrrolidons (wie beispielsweise Luviskol^{®} K 90 oder Luviskol^{®} K 85 der Firma BASF SE), Copolymerisate aus Vinylpyrrolidon und Vinylacetat (wie sie beispielsweise unter dem Warenzeichen Luviskol^{®} VA 37, Luviskol^{®} VA 55, Luviskol^{®} VA 64 und Luviskol^{®} VA 73 von der Firme BASF SE vertrieben werden), Terpolymere aus Vinylpyrrolidon, Vinylacetat und Vinylpropionat, Polyacrylamide (wie beispielsweise Akypomine^{®} P 191 von der Firma CHEM-Y), Polyvinylalkohole (die beispielsweise unter den Handelsbezeichnungen Elvanol^{®} von Du Pont oder Vinol^{®} 523/540 von der Firma Air Products vertrieben werden), Terpolymere aus Vinylpyrrolidon, Methacrylamid und Vinylimidazol (wie beispielsweise Luviset^{®} Clear der Firma BASF SE).

Neben den auf ethylenisch ungesättigten Monomeren basierenden nichtionischen Polymeren eignen sich weiterhin zur bevorzugten Ausführung der technischen Lehre nichtionische Cellulosederivate als filmbildende und/oder festigende Polymere, die bevorzugt ausgewählt werden aus Methylcellulose und insbesondere aus Celluloseether, wie Hydroxypropylcellulose (z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird), Hydroxyethylcellulose, wie sie beispielsweise unter den Warenzeichen Culminal^{®} und Benecel^{®} (AQUALON) und Natrosol^{®}-Typen (Hercules) vertrieben werden.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Copolymere von quaternierten Derivaten des Dialkylaminoalkyl(meth)acrylats und/oder Copolymere von quaternierten Derivaten des Dialkylaminoalkyl(meth)acrylamids gelten als besonders bevorzugt geeignete kationische filmbildende und/oder kationische festigende Polymere.

Copolymere mit Monomereinheiten gemäß Formel (M1) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Ein weiterhin erfindungsgemäß bevorzugt geeignetes kationisches filmbildendes und/oder kationisches festigendes Polymer ist mindestens ein kationisches filmbildendes und/oder kationisches festigendes Polymer, das mindestens ein Strukturelement der Formel (M9) und zusätzlich mindestens ein Strukturelement der Formel (M10) enthält 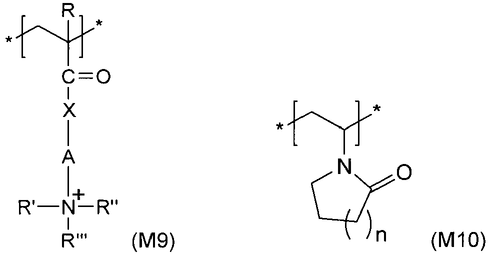 worin
- R: für ein Wasserstoffatom oder eine Methylgruppe steht,
- R', R" und R": unabhängig voneinander stehen für eine (C₁ bis C₃₀)-Alkylgruppe,
- X: steht für ein Sauerstoffatom oder eine Gruppe NH,
- A: steht für eine Ethan-1,2,-diylgruppe oder eine Propan-1,3-diylgruppe,
- n: 1 oder 3 bedeutet.

Zur Kompensation der positiven Polymerladung dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Solche Verbindungen sind beispielsweise als
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE).

Weiterhin werden die kationischen filmbildenden und/oder kationischen festigenden Polymere erfindungsgemäß besonders bevorzugt aus kationischen, quaternisierten Cellulose-Derivaten ausgewählt.

Ferner eignen sich als filmbildendes und/oder festigendes Polymere bevorzugt kationische, quaternisierte Cellulosederivate.

Es erweisen sich solche kationischen, quaternisierten Cellulosen als im Sinne der Erfindung besonders vorteilhaft, die in einer Seitenkette mehr als eine permanente kationische Ladung tragen. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Als im Sinne der Erfindung besonders bevorzugt verwendbare kationische Polymere dienen weiterhin solche kationischen filmbildenden und/oder kationischen festigenden Copolymere, die mindestens ein Strukturelement der Formel (M11) 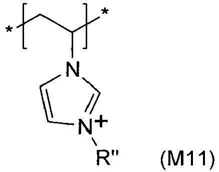 worin
R" für eine (C₁ bis C₄)-Alkylgruppe, insbesondere eine Methylgruppe, steht,
und zusätzlich mindestens ein weiteres kationisches und/oder nichtionisches Strukturelement aufweisen.

Zur Kompensation der positiven Polymerladung dienen alle möglichen physiologisch verträglichen Anionen, wie beispielsweise Chlorid, Bromid, Hydrogensulfat, Methylsulfat, Ethylsulfat,-Tetrafluoroborat, Phosphat, Hydrogenphosphat, Dihydrogenphosphat oder p-Toluolsulfonat, Triflat.

Es ist wiederum erfindungsgemäß bevorzugt, wenn als zusätzliches kationisches filmbildendes und/oder kationisches festigendes Polymer, mindestens ein Copolymer (c1) enthalten ist, das neben mindestens einem Strukturelement der Formel (M11) zusätzlich ein Strukturelement der Formel (M6) umfasst 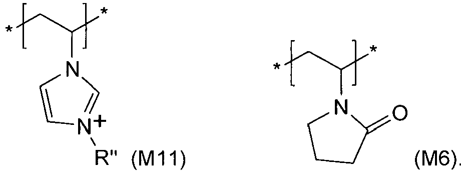 worin
R" für eine (C₁ bis C₄)-Alkylgruppe, insbesondere eine Methylgruppe, steht.

Zur Kompensation der positiven Polymerladung der Copolymere (c1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (c1) enthalten 10 bis 30 Mol-%, vorzugsweise 15 bis 25 Mol.-% und insbesondere 20 Mol.-% Struktureinheiten gemäß Formel (M11) und 70 bis 90 Mol.-%, vorzugsweise 75 bis 85 Mol.-% und insbesondere 80 Mol.-% Struktureinheiten gemäß Formel (M6).

Hierbei ist besonders bevorzugt, wenn die Copolymere (c1) neben Polymereinheiten, die aus dem Einbau der genannten Struktureinheiten gemäß Formel (M11) und (M6) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c1) ausschließlich aus Struktureinheiten der Formel (M11) mit R" = Methyl und (M6) aufgebaut und lassen sich durch die allgemeine Formel (Poly1) 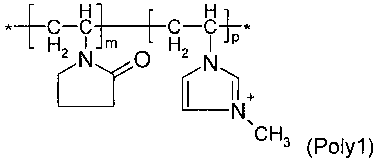 beschreiben, wobei die Indices m und p je nach Molmasse des Polymers variieren und nicht bedeuten sollen, daß es sich um Blockcopolymere handelt. Vielmehr können Struktureinheiten der Formel (M11) und der Formel (M6) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Besonders bevorzugte erfindungsgemäße Mittel enthalten ein Copolymer (c1), insbesondere der Formel (Poly1), das Molmassen innerhalb eines bestimmten Bereiches aufweist. Hier sind erfindungsgemäße Mittel bevorzugt, bei denen das Copolymer (c1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (c1) oder an dessen bzw. deren Stelle können die erfindungsgemäßen Mittel auch Copolymere (c2) enthalten, die ausgehend vom Copolymer (c1) als zusätzliche Struktureinheiten Struktureinheiten der Formel (M7) aufweisen

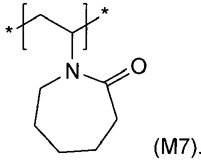

Weitere besonders bevorzugte erfindungsgemäße Mittel sind somit dadurch gekennzeichnet, daß sie als kationisches filmbildendes und/oder kationisches festigendes Polymer mindestens ein Copolymer (c2) enthalten, das mindestens eine Struktureinheit gemäß Formel (M11-a) und mindestens eine Struktureinheit gemäß Formel (M6) und mindestens eine Struktureinheit gemäß Formel (M7) enthält

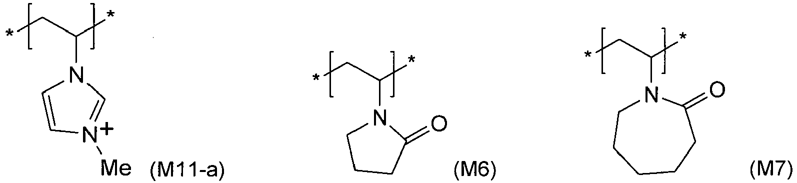

Auch hierbei ist besonders bevorzugt, wenn die Copolymere (c2) neben Polymereinheiten, die aus dem Einbau der genannten Struktureinheiten gemäß Formel (M11-a), (M6) und (M7) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c2) ausschließlich aus Struktureinheiten der Formeln (M11-a), (M6) und (M7) aufgebaut und lassen sich durch die allgemeine Formel (Poly2) 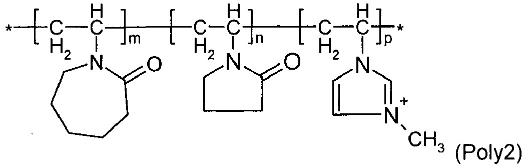 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente (c2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (c2) enthalten 1 bis 20 Mol-%, vorzugsweise 5 bis 15 Mol.-% und insbesondere 10 Mol.-% Struktureinheiten gemäß Formel (M11-a) und 30 bis 50 Mol.-%, vorzugsweise 35 bis 45 Mol.-% und insbesondere 40 Mol.-% Struktureinheiten gemäß Formel (M6) und 40 bis 60 Mol.-%, vorzugsweise 45 bis 55 Mol.-% und insbesondere 60 Mol.-% Struktureinheiten gemäß Formel (M7).

Besonders bevorzugte erfindungsgemäße Mittel enthalten ein Copolymer (c2), das Molmassen innerhalb eines bestimmten Bereiches aufweist. Hier sind erfindungsgemäße Mittel bevorzugt, bei denen das Copolymer (c2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (c1) und/oder (c2) oder an dessen bzw. deren Stelle können die erfindungsgemäßen Mittel als filmbildendes kationische und/oder festigendes kationisches Polymer auch Copolymere (c3) enthalten, die als Struktureinheiten Struktureinheiten der Formeln (M11-a) und (M6) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als zusätzliches kationisches filmbildendes und/oder kationisches festigendes Polymer mindestens ein Copolymer (c3) enthalten, das mindestens eine Struktureinheit gemäß Formel (M11-a) und mindestens eine Struktureinheit gemäß Formel (M6) und mindestens eine Struktureinheit gemäß Formel (M10) und mindestens eine Struktureinheit gemäß Formel (M12) enthält

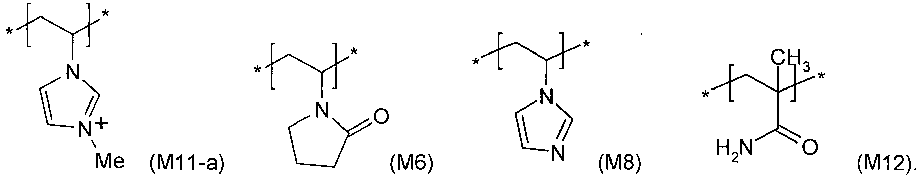

Auch hierbei ist besonders bevorzugt, wenn die Copolymere (c3) neben Polymereinheiten, die aus dem Einbau der genannten Struktureinheiten gemäß Formel (M11-a), (M6), (M8) und (M12) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (c3) ausschließlich aus Struktureinheiten der Formel (M11-a), (M6), (M8) und (M12) aufgebaut und lassen sich durch die allgemeine Formel (Poly3) 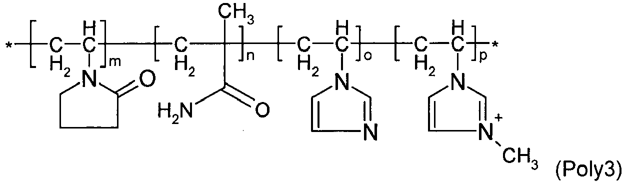 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M11-a), (M6), (M8) und (M12) im Molekül statistisch verteilt vorliegen.

Zur Kompensation der positiven Polymerladung der Komponente (c3) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (M11-a) und 45 bis 65 Mol.-%, vorzugsweise 50 bis 60 Mol.-% und insbesondere 55 Mol.-% Struktureinheiten gemäß Formel (M6) und 1 bis 20 Mol.-%, vorzugsweise 5 bis 15 Mol.-% und insbesondere 10 Mol.-% Struktureinheiten gemäß Formel (M8) und 20 bis 40 Mol.-%, vorzugsweise 25 bis 35 Mol.-% und insbesondere 29 Mol.-% Struktureinheiten gemäß Formel (M12).

Besonders bevorzugte erfindungsgemäße Mittel enthalten ein Copolymer (c3), das Molmassen innerhalb eines bestimmten Bereiches aufweist. Hier sind erfindungsgemäße Mittel bevorzugt, bei denen das Copolymer (c3) eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen filmbildenden kationischen und/oder festigenden Polymer ausgewählt aus den kationischen Polymeren mit mindesten einem Strukturelement der obigen Formel (M11-a), gelten als bevorzugt:
Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat^{®} FC 370, Luviquat^{®} FC 550, Luviquat^{®} FC 905 und Luviquat^{®} HM 552 (BASF SE)), Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)), Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
sowie Gemische aus diesen Polymeren.

Weitere in den erfindungsgemäßen Mitteln bevorzugt einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Zu diesen Polymeren gehören beispielsweise Chitosane. Chitosan und/oder Chitosanderivate gelten als ganz besonders bevorzugt geeignete filmbildende und/oder festigende Polymere im Sinne der vorliegenden Erfindung.

Chitosane stellen Biopolymere dar und werden zur Gruppe der Hydrokolloide gezählt. Chemisch betrachtet, handelt es sich um partiell deacetylierte Chitine unterschiedlichen Molekulargewichtes.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineral-säuren demineralisiert und schließlich durch Zugabe von starken Basen deacetyliert, wobei die Molekulargewichte über ein breites Spektrum verteilt sein können. Vorzugsweise werden solche Typen eingesetzt, die ein durchschnittliches Molekulargewicht von 800.000 bis 1.200.000 Dalton, eine Viskosität nach Brook-field (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen.

Neben den Chitosanen als typischen kationischen Biopolymeren kommen im Sinne der Erfindung auch kationisch derivatisierte Chitosane (wie z. B. Quaternierungsprodukte) oder alkoxylierte Chitosane in Frage.

Erfindungsgemäß bevorzugte Mittel sind dadurch gekennzeichnet, daß sie als Chitosanderivat(e) Neutralisationsprodukte von Chitosan mit mindestens einer Säure, ausgewählt aus Milchsäure, Pyrrolidoncarbonsäure, Nicotinsäure, Hydroxyisobuttersäure, Hydroxyisovaleriansäure enthaltend oder Gemische dieser Neutralisationsprodukte umfassen.

Geeignete Chitosan(derivate) sind beispielsweise unter den Handelsbezeichnungen Hydagen^{®} CMF (1 Gew.-% Aktivsubstanz in wässriger Lösung mit 0.4 Gew.-% Glykolsäure, Molekulargewicht 500000 bis 5000000 g/mol Cognis), Hydagen^{®} HCMF (Chitosan (zu 80 % deacetyliert), Molekulargewicht 50000 bis 1000000 g/mol, Cognis), Kytamer^{®} PC (80 Gew.-% Aktivsubstanz an Chitosan pyrolidoncarboxylat (INCI-Bezeichnung: Chitosan PCA), Amerchol) und Chitolam^{®} NB/101 im Handel frei verfügbar.

Das Chitosan bzw. dessen Derivate sind in den erfindungsgemäßen Mitteln bevorzugt in einer Menge von 0,01 Gew.-% bis 20 Gew.-%, besonders bevorzugt von 0,01 Gew.-% bis 10,0 Gew.-%, ganz besonders bevorzugt von 0,1 Gew.-% bis 1 Gew.-%, jeweils bezogen auf das Gewicht des erfindungsgemäßen Mittels, enthalten.

Als im Sinne der Erfindung bevorzugt geeignete temporär kationische Polymere gelten gleichfalls solche, die mindestens eine Struktureinheit der Formeln (M1-1) bis (M1-8) aufweisen

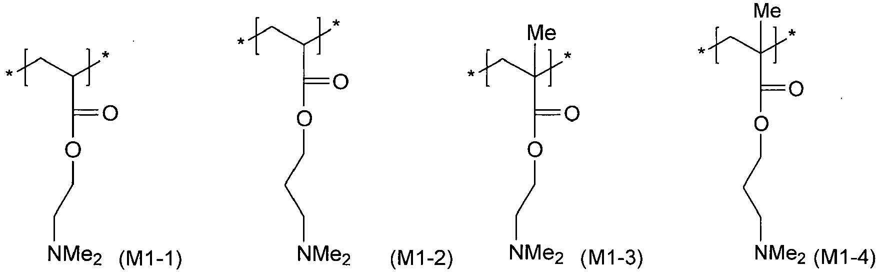

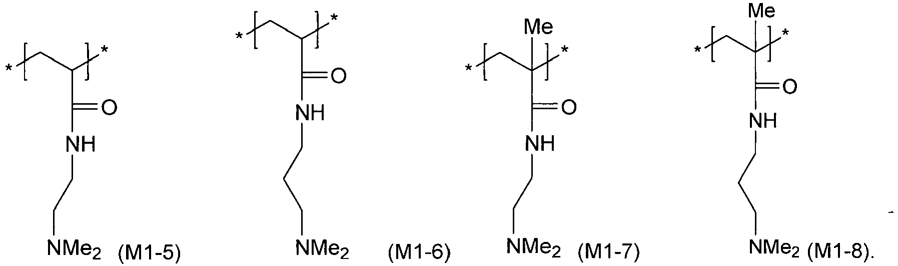

Dabei sind wiederum solche Copolymere bevorzugt, die mindestens eine Struktureinheit der Formeln (M1-1) bis (M1-8) und zusätzlich mindestens eine Struktureinheit der Formel (M10) enthalten, 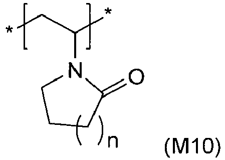 worin
- n: 1 oder 3 bedeutet.

Dabei gilt wiederum die Gruppe der Polymere
- N-Vinylpyrrolidon/N-Vinylcaprolactam/Dimethylaminopropylmethacrylamid-Copolymer (beispielsweise INCI-Bezeichnung: VP/Vinyl Caprolactam/DMAPA Acrylates Copolymer unter dem Handelsnamen Aquaflex^{®} SF-40 (ISP)),
- N-Vinylcaprolactam/N-Vinylpyrrolidon/Dimethylaminoethylmethacrylat-Copolymer (beispielsweise als.35-39% Festkörper in Ethanol in form des Handelsprodukts Advantage LC E mit der INCI-Bezeichnung: Vinyl Caprolactam/VP/Dimethylaminoethyl Methacrylate Copolymer, Alcohol, Lauryl Pyrrolidone (ISP)),
- N-Vinylpyrrolidon/Dimethylaminopropylmethacrylamid-Copolymer (beispielsweise INCI-Bezeichnung: VP/DMAPA Acrylates Copolymer unter dem Handelsnamen Styleze CC-10 (ISP)),
als bevorzugte Liste zur Auswahl.

Die erfindungsgemäßen Mittel können als filmbildendes und/oder festigendes Polymer auch mindestens ein amphoteres Polymer enthalten. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt.

Letztere weisen zusätzlich zu der kationogenen Gruppe bzw. positiv geladenen Gruppe mindestens eine negativ geladene Gruppe im Molekül auf und werden auch als zwitterionische Polymere bezeichnet.

Die amphoteren Polymere sind in den erfindungsgemäßen Mitteln bevorzugt in Mengen von 0,01 Gew.-% bis 20 Gew.-%, besonders bevorzugt von 0,05 bis 10 Gew.-%, bezogen auf das gesamte Mittel, enthalten. Mengen von 0,1 bis 5 Gew.-% sind ganz besonders bevorzugt.

Weiterhin können als filmbildende und/oder festigende Polymere mindestens ein anionisches filmbildendes und/oder anionisches festigendes Polymer eingesetzt werden.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Weitere bevorzugt einsetzbare anionische Polymere werden ausgewählt aus der Gruppe, die gebildet wird, aus
- Copolymeren aus Vinylacetat und Crotonsäure (wie sie beispielsweise als Handelsprodukt Aristoflex^{®} A 60 mit der INCI-Bezeichnung VA/Crotonates Copolymer von der Firma CIBA in einer 60 Gew.-%-igen Dispersion in Isopropanol-Wasser vermarktet werden),
- Copolymeren aus Ethylacrylat und Methacrylsäure (wie sie beispielsweise unter dem Handelsnamen Luviflex^{®} Soft mit einer Säurezahl von 84 bis 105 unter der INCI-Bezeichnung Acrylates Copolymer in einer ca. 20 bis 30 Gew.-%igen Dispersion in Wasser von der Firma BASF SE vertrieben werden),
- Polyurethanen mit mindestens einer Carboxylgruppe (wie beispielsweise ein Copolymer aus Isophthalsäure, Adipinsäure, 1,6-Hexandiol, Neopentylglykol und Isophorondiisocyanat wie es unter dem Handelsnamen Luviset PUR mit der INCI-Bezeichung Polyurethane-1 von der Firma BASF SE vertrieben wird).

Falls insbesondere stark verdickend wirkende anionische Polymere zum Einsatz kommen, sollte wiederum im Rahmen einer bevorzugten Ausführungsform darauf geachtet werden, dass das zuvor genannte bevorzugte Viskositätskriterium der erfindungsgemäßen Mittel eingehalten wird.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Zur Intensivierung des erfindungsgemäßen Effektes enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin^{®} CS 15 (COGNIS), Cremophor A25 (BASF SE) bzw. Eumulgin^{®} CS 50 (COGNIS) vermarktet werden.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Erfindungsgemäß einsetzbar sind weiterhin kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈- C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Die erfindungsgemäßen Mittel enthalten die Inhalts- bzw- Wirkstoffe in einem kosmetisch akzeptablen Träger.
Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Es ist erfindungsgemäß bevorzugt mindestens einen (C₁ bis C₄)-Monoalkylalkohol in den erfindungsgemäßen Mitteln insbesondere in einer Menge von 1 bis 50 Gew.-% insbesondere von 5 bis 30 Gew.-% einzusetzen. Dies ist wiederum insbesondere für die Konfektionierung als Pumpschaum oder Aerosolschaum bevorzugt.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole verstanden. Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401 DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein.

Dimethiconcopolyole (S3) bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning^{®} 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäß Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning^{®} 939 oder als Handelsprodukt Dow Corning^{®} 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben.

Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die Mittel enthalten die Vitamin A-Komponente bevorzugt in Mengen von 0,05-1 Gew.-%, bezogen auf die gesamte Anwendungszubereitung.

Zur Vitamin B-Gruppe oder zu dem Vitamin B-Komplex gehören u. a. Vitamin B₁ (Thiamin), Vitamin B₂ (Riboflavin), Vitamin B₃ (Nicotinsäure und/oder Nicotinsäureamid (Niacinamid)), Vitamin B₅ (Pantothensäure, Panthenol und Pantolacton), Vitamin B₆ (Pyridoxin sowie Pyridoxamin und Pyridoxal), Vitamin C (Ascorbinsäure), Vitamin E (Tocopherole, insbesondere α-Tocopherol), Vitamin F (Linolsäure und/oder Linolensäure), Vitamin H.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt.

Ganz besonders bevorzugt wird als Pflegestoff D-Panthenol, gegebenenfalls in Kombination mit mindestens einem der oben genannten Silikonderivate eingesetzt.

Wie auch der Zusatz von Glycerin und/oder Propylenglykol erhöht der Zusatz von Panthenol die Flexibilität des bei Anwendung des erfindungsgemäßen Mittels gebildeten Polymerfilms. Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Weiterhin kann es bevorzugt sein, in den erfindungsgemäßen Mitteln Mischungen aus mehreren, insbesondere aus zwei, verschiedenen Pflanzenextrakten einzusetzen.

Auch Mono- bzw. Oligosaccharide können als Pflegestoff in den erfindungsgemäßen Mitteln eingesetzt werden.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit.

Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 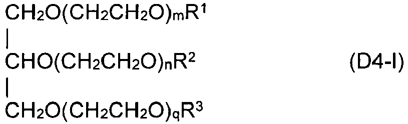 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbare UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxy-benzophenon-5-sulfonsäure und deren Natriumsalz (Benzophenone-4; Uvinul^{®}MS 40; Uvasorb^{®}S 5), 3-(4'-Methylbenzyliden)-D,L-Campher, 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon, 1,1'-Diphenylacrylonitrilsäure-2-ethylhexyl-ester, o-Aminobenzoesäure-menthylester, 2,2',4,4'-Tetrahydroxybenzophenon, 2,2'-Dihydroxy-4,4'-dimethoxybenzophenon, 2,2'-Dihydroxy-4,4'-dimethoxybenzophenon-5-natriumsulfonat und 2-Cyano-3,3-diphenylacrylsäure-2'-ethylhexylester. Bevorzugt sind 2-Hydroxy-4-methoxy-benzophenon-5-sulfonsäure und deren Natriumsalz und/oder ethoxylierte 4-Aminobenzoesäure-ethylester.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Farbstoffe, die auch unter den Bezeichnungen Basic Yellow 87, Basic Orange 31 und Basic Red 51 bekannt sind, sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Vorzugsweise liegen im Rahmen einer weiteren Ausführungsform die erfindungsgemäßen Mittel in Form einer Creme oder eines Gels, insbesondere als Gel, vor.

Dabei ist es wiederum bevorzugt, wenn in die erfindungsgemäße Creme bzw. das erfindungsgemäße Gel Gasblasen eingearbeitet sind. Diese Gasblasen sind für das menschliche Auge sichtbar. Als Gase eignen sich beispielsweise Luft, Stickstoff, Sauerstoff, Kohlendioxyd, Distickstoffmonoxyd, Argon.

Die creme- bzw. gelförmigen erfindungsgemäßen Mittel weisen bevorzugt eine Viskosität von 10000 bis 500000 mPas, besonders bevorzugt von 30000 bis 300000 mPas, (jeweils gemessen mit Brookfield RVDV II+ mit Heilpath, Spindel T-E, 5 rpm, 20°C) auf.

Liegt das erfindungsgemäße Mittel in Form eines Gels vor, so handelt es sich besonders bevorzugt um ein transparentes Gel.

Ein zweiter Gegenstand der Erfindung ist die Verwendung der erfindungsgemäßen Mittel zur temporären Verformung von Haaren und/oder zur Haarpflege.

Die erfindungsgemäßen Mittel und Produkte, die diese Mittel enthalten, insbesondere Haargele oder Haarcremes, zeichnen sich insbesondere dadurch aus, dass sie dem behandelten Haar einen sehr starken, dauerhaften Frisurenhalt verleihen und das Haar flexibel bleibt. Wird das Mittel als Haargel konfektioniert, resultiert ein Gel mit einer teigigen Konsistenz, das sich dennoch gleichmäßig und ohne zu tropfen auf dem Haar verteilen lässt.

Es ist erfindungsgemäß bevorzugt, das Mittel des ersten Erfindungsgegenstandes als leave-on Haarbehandlungsmittel zu verwenden.

Ein dritter Gegenstand der Erfindung ist ein Verfahren zur Behandlung keratinhaltiger Fasern, insbesondere menschlicher Haare, worin das erfindungsgemäße Mittel des ersten Erfindungsgegenstandes auf die keratinhaltigen Fasern appliziert wird.

Es ist erfindungsgemäß bevorzugt, wenn die keratinhaltigen Fasern vor, während oder nach der Applikation des erfindungsgemäßen Mittels in Form gebracht werden.

Ferner gilt es erfindungsgemäß als bevorzugt, im Rahmen des erfindungsgemäßen Verfahrens das erfindungsgemäße Mittel nicht aus den keratinhaltigen Fasern auszuspülen.

Die nachfolgenden Beispiele sollen den Gegenstand der vorliegenden Erfindung erläutern ohne ihn in irgendeiner Weise zu beschränken.

### Beispiele

Die folgenden Mengenangaben verstehen sich - soweit nichts anderes vermerkt ist - in Gewichtsprozent.

### 1.0 Rezepturen

Es wurden die Stylinggele A bis E gemäß folgender Tabelle hergestellt.

| | A | B | C | D | E |
|---|---|---|---|---|---|
| Rohstoffe | Vergleich | | | | erfindungsgemäß |
| Benzophenone-4 | 0,05 | 0,05 | - | - | - |
| Synthalen K ¹ | 0,40 | - | - | - | - |
| Neolone PE ² | 0,50 | 0,50 | - | - | - |
| Dinatrium EDTA | 0,05 | 0,05 | 0,05 | 0,05 | 0,05 |
| Sorbitol | 3,00 | 3,00 | 3,00 | 3,00 | 3,00 |
| D-Panthenol | 0,15 | 0,15 | 0,15 | 0,15 | 0,15 |
| Neutrol TE ³ | 0,50 | 0,50 | - | - | - |
| Aquastyle^{®} 300 ⁴ | 10,00 | 10,00 | 7,00 | 9,00 | 7,00 |
| Aculyn 28 ⁵ | - | - | 6,00 | 6,00 | - |
| Aculyn 88 ⁶ | - | - | - | - | 6,00 |
| Luviskol VA 64 W ⁷ | - | - | 5,00 | - | 5,00 |
| PEG-40 Hydrogenated Castor Oil | 0,55 | 0,55 | 0,55 | 0,55 | 0,55 |
| Uvinul P 25 ⁸ | - | - | 0,10 | 0,10 | 0,10 |
| 2-Phenoxyethanol | - | - | 0,60 | 0,60 | 0,60 |
| Carbopol Ultrez 21 ⁹ | - | 0,30 | - | - | - |
| 2-Amino-2-methylpropan-1-ol | - | - | 0,40 | 0,40 | 0,40 |
| Parfum | 0,10 | 0,10 | 0,10 | 0,10 | 0,10 |
| Wasser | ad 100 | ad 100 | ad 100 | ad 100 | ad 100 |

| | | | | | |
|---|---|---|---|---|---|
| ¹ Polyacrylsäure (ca. 89% Aktivsubstanzgehalt; INCI-Bezeichnung: Carbomer) (3V Sigma) ² Lösung von ca. 2 Gew.-% 2-Methyl-4-isothiazolin-3-on in ca. 83.5 Gew.-% Phenoxyethanol, 12.5 Gew.-% Propan-1,2-diol und ca. 2 Gew.-% Wasser (INCI-Bezeichnung: Phenoxyethanol, Methylisothiazolinone) (Rohm & Haas), ³ N,N,N',N',-Tetrakis-(2-hydroxypropyl)-ethylendiamin (INCI-Bezeichnung: Tetrahydroxypropyl Ethylenediamine) (BASF) ⁴ Copolymer aus N-Vinylpyrrolidon/N-Vinylcaprolactam/N-(3-Dimethylaminopropyl)methacrylamid und 3-(Methacryloylamino)propyl-lauryldimethylammoniumchlorid (Aktivsubstanz 30 Gew.-% in Wasser/Ethanol, INCI-Bezeichnung: Polyquaternium-69) (ISP), ⁵ Copolymer aus (Meth)acrylsäure, (Meth)acrylsäureester und Beheneth-25-Methacrylsäureester (19-21 Gew.-% Festkörper in Wasser; INCI-Bezeichnung: Acrylates/Beheneth-25 Methacrylate Copolymer) (Rohm und Haas), ⁶ vernetztes, amphiphiles, anionisches Polymer (28 bis 30 Gew.-%-ige Dispersion in Wasser, INCI-Bezeichnung Acrylates / Steareth-20 Methacrylate Crosspolymer) (Rohm & Haas) ⁷ Copolymer aus Vinylacetat und N-Vinylpyrrolidon im Verhältnis 40 zu 60 (50 % Aktivsubstanz in Wasser, INCI-Bezeichnung: VP/VA Copolymer) (BASF) ⁸ 1,4-Ethoxylated (25 EO) aminobenzoesäureethylester (INCI-Bezeichnung: PEG25 PABA) (BASF) ⁹ vernetztes Acrylsäurecopolymer, weißes Pulver (INCI-Bezeichnung: Acrylates / C10-30 Alkylacrylate Crosspolymer) (Noveon), | | | | | |

Die Vergleichsrezepturen A und B waren eingetrübt.

Die erfindungsgemäße Rezeptur E lag als klares, transparentes Gel vor. Luftblasen konnten lagerstabil eingearbeitet werden. Bei Anwendung auf menschlichem Haar ließ sich hervorragende Formstabilisierung erzielen.

## Patentansprüche

1. Mittel zur Behandlung keratinhaltiger Fasern, insbesondere menschlichem Haar, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 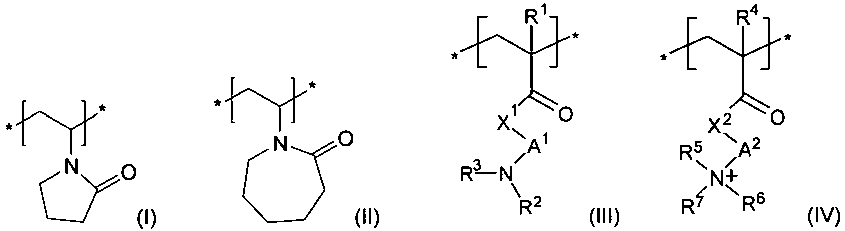 worin
R¹ und R⁴ unabhängig voneinander stehen für ein Wasserstoffatom oder eine Methylgruppe,
X¹ und X² unabhängig voneinander stehen für ein Sauerstoffatom oder eine Gruppe NH,
A¹ und A² unabhängig voneinander stehen für eine Gruppe Ethan-1,2-diyl, Propan-1,3-diyl oder Butan-1,4-diyl,
R², R³, R⁵ und R⁶ unabhängig voneinander stehen für eine (C₁ bis C₄)-Alkylgruppe,
R⁷ steht für eine (C₈ bis C₃₀)-Alkylgruppe
und
(b) mindestens ein vernetztes amphiphiles, anionisches Polymer, umfassend mindestens eine Struktureinheit der Formel (V) und mindestens eine Struktureinheit der Formel (VI), 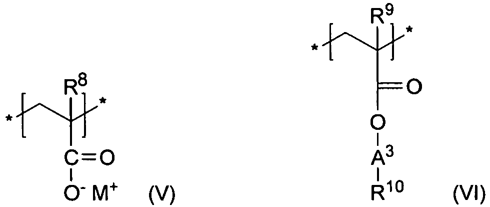 worin
R⁸ und R⁹ stehen unabhängig voneinander für ein Wasserstoffatom oder eine Methylgruppe,
R¹⁰ steht für eine (C₈ bis C₃₀)-Alkylgruppe,
M⁺ steht für ein äquivalent eines physiologisch verträglichen Kations und
A³ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe *-(CH₂CH₂O)ₓ-(CH₂CHMeO)_{y}-* worin die Summe x + y für eine ganze Zahl von 5 bis 35 steht und x und y größer als Null sind.

2. Mittel nach Anspruch 1, **dadurch gekennzeichnet, dass** A¹ und A² stehen unabhängig voneinander für Ethan-1,2-diyl oder Propan-1,3-diyl.

3. Mittel nach einem der Ansprüche 1 oder 2, **dadurch gekennzeichnet, dass** R², R³, R⁵ und R⁶ stehen unabhängig voneinander für Methyl oder Ethyl, insbesondere für Methyl.

4. Mittel nach einem der Ansprüche 1 bis 3, **dadurch gekennzeichnet, dass** R⁷ steht für Decyl, Undecyl, Dodecyl, Tridecyl, Tetradecyl, Hexadecyl, Octadecyl, Eicosyl oder Docosyl.

5. Mittel nach einem der Ansprüche 1 bis 4, **dadurch gekennzeichnet, dass** das amphiphile, kationische Polymer ausgewählt wird, aus mindestens einem Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 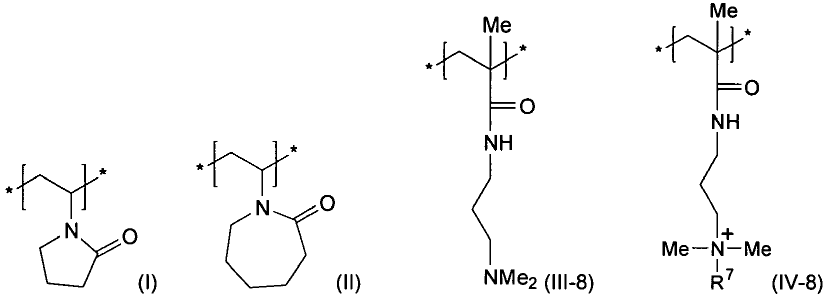 worin R⁷ steht für eine (C₈ bis C₃₀)-Alkylgruppe.

6. Mittel nach einem der Ansprüche 1 bis 5, **dadurch gekennzeichnet, dass** es die amphiphilen, kationischen Polymere (a) in einer Menge von 0,05 Gew.-% bis 15,0 Gew.-%, besonders bevorzugt von 0,05 Gew.-% bis 10,0 Gew.-%, ganz besonders bevorzugt von 0,1 bis 5,0 Gew.%, jeweils bezogen auf das Gewicht des Mittels, enthält.

7. Mittel nach einem der Ansprüche 1 bis 6, **dadurch gekennzeichnet, dass** es die amphiphilen, anionischen Polymere (b) in einer Menge von 0,05 Gew.-% bis 10,0 Gew.-%, besonders bevorzugt von 0,05 Gew.-% bis 5,0 Gew.-%, ganz besonders bevorzugt von 0,1 bis 2,0 Gew.-% jeweils bezogen auf das Gewicht des Mittels, enthält.

8. Mittel nach einem der Ansprüche 1 bis 7, **dadurch gekennzeichnet, dass** die amphiphilen, kationischen Polymere (a) und die amphiphilen, anionischen Polymere (b) in einem Gewichtsverhältnis von 1 zu 10 bis 10 zu 1, insbesondere von 1 zu 5 bis 5 zu 1, ganz besonders bevorzugt von 1 zu 3.5 bis 3.5 zu 1, eingesetzt werden.

9. Mittel nach einem der Ansprüche 1 bis 8, **dadurch gekennzeichnet, dass** die amphiphilen, anionischen Polymere (b) aus der Gruppe der Copolymere b1 ausgewählt werden, die mindestens eine Struktureinheit der Formel (V-1), mindestens eine Struktureinheit der Formel (V-2) sowie mindestens eine Struktureinheit der Formel (VI) umfassen 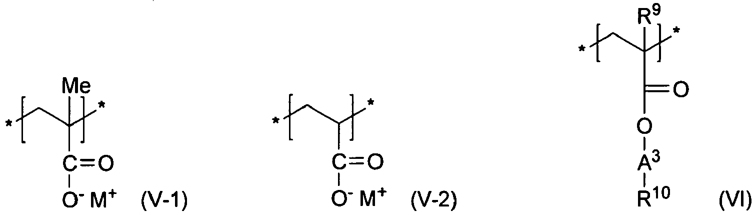 worin
M⁺ unabhängig voneinander für ein physiologisch verträgliches Kation steht,
R⁹ steht für ein Wasserstoffatom oder eine Methylgruppe,
R¹⁰ steht für eine (C₈ bis C₃₀)-Alkylgruppe,
A³ steht für eine Gruppe *-(CH₂CH₂O)ₓ-* worin x für eine ganze Zahl von 5 bis 35 steht, eine Gruppe *-(CH₂CHMeO)_{y}-* worin y für eine ganze Zahl von 5 bis 35 steht oder eine Gruppe *-(CH₂CH₂O)ₓ-(CH₂CHMeO)_{y}-* worin die Summe x + y für eine ganze Zahl von 5 bis 35 steht und x und y größer als Null sind.

10. Mittel nach einem der Ansprüche 1 bis 9, **dadurch gekennzeichnet, dass** die amphiphilen, anionischen Polymere (b) aus Formel (b1-1) ausgewählt werden, 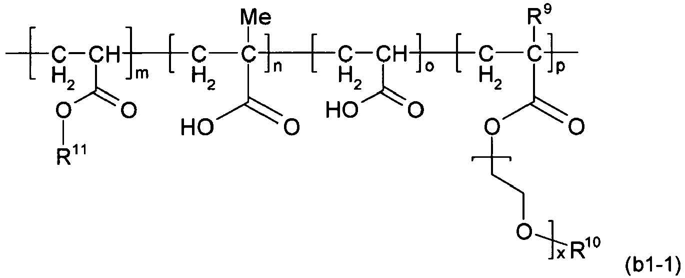 wobei
die Indices m, n, o und p jeweils eine ganze Zahl größer Null bedeuten,
R⁹ steht für ein Wasserstoffatom oder eine Methylgruppe,
R¹⁰ steht für einen Kohlenwasserstoffrest mit 8 bis 30 Kohlenstoffatomen,
R¹¹ steht für eine (C₁ bis C₄)-Alkylgruppe,
x steht für 5 bis 35.

11. Mittel nach einem der Ansprüche 1 bis 10, **dadurch gekennzeichnet, dass** zusätzlich mindestens ein filmbildendes und/oder festigendes Polymer enthalten ist.

12. Mittel nach einem der Ansprüche 1 bis 11, **dadurch gekennzeichnet, dass** zusätzlich mindestens ein Tensid, insbesondere mindestens ein nichtionisches Tensid, enthalten ist.

13. Mittel nach einem der Ansprüche 1 bis 12, **dadurch gekennzeichnet, dass** es zusätzlich mindestens ein Alkanolamin enthält.

14. Verfahren zur Behandlung keratinhaltiger Fasern, insbesondere menschlicher Haare, worin ein Mittel gemäß wenigstens eines der Ansprüche 1 bis 13 auf die keratinhaltigen Fasern appliziert wird.

## Claims

1. An agent for treating keratin-containing fibers, in particular human hair, containing in a cosmetically acceptable carrier:
(a) at least one amphiphilic, cationic polymer having at least one structural unit of formula (I), at least one structural unit of formula (II), at least one structural unit of formula (III), and at least one structural unit of formula (IV), 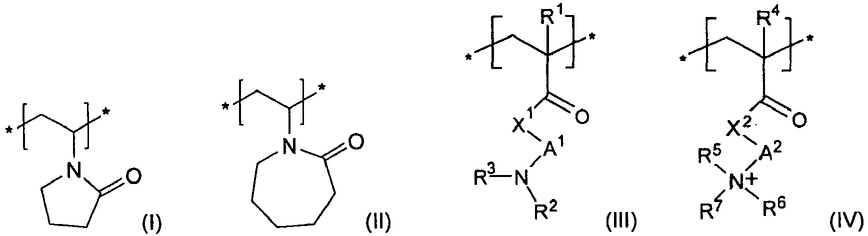 where
R¹ and R⁴ represent, independently of one another, a hydrogen atom or a methyl group,
X¹ and X² represent, independently of one another, an oxygen atom or an NH group,
A¹ and A² represent, independently of one another, an ethane-1,2-diyl, propane-1,3-diyl, or butane-1,4-diyl group,
R², R³, R⁵ and R⁶ represent, independently of one another, a (C₁ to C₄) alkyl group,
R⁷ represents a (C₈ to C₃₀) alkyl group,
and
(b) at least one crosslinked amphiphilic, anionic polymer having at least one structural unit of formula (V) and at least one structural unit of formula (VI), 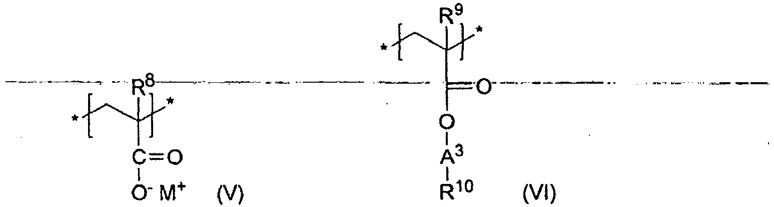 where
R⁸ and R⁹ represent, independently of one another, a hydrogen atom or a methyl group,
R¹⁰ represents a (C₈ to C₃₀) alkyl group,
M⁺ represents an equivalent of a physiologically acceptable cation, and
A³ represents a *-(CH₂CH₂O)x-* group, where x represents an integer from 5 to 35,
a *-(CH₂CHMeO)_{y}-* group, where y represents an integer from 5 to 35, or a *-(CH₂CH₂O)ₓ-(CH₂CHMeO)_{y}-* group, where the sum of x + y represents an integer from 5 to 35, and x and y are greater than zero.

2. The agent according to claim 1, **characterized in that** A¹ and A² represent, independently of one another, ethane-1,2-diyl or propane-1,3-diyl.

3. The agent according to one of claims 1 or 2, **characterized in that** R², R³, R⁵ and R⁶ represent, independently of one another, methyl or ethyl, in particular methyl.

4. The agent according to one of claims 1 to 3, **characterized in that** R⁷ represents decyl, undecyl, dodecyl, tridecyl, tetradecyl, hexadecyl, octadecyl, eicosyl or docosyl.

5. The agent according to one of claims 1 to 4, **characterized in that** the amphiphilic, cationic polymer is selected from at least one polymer having at least one structural unit of formula (I), at least one structural unit of formula (II), at least one structural unit of formula (III-8) and at least one structural unit of formula (IV-8), 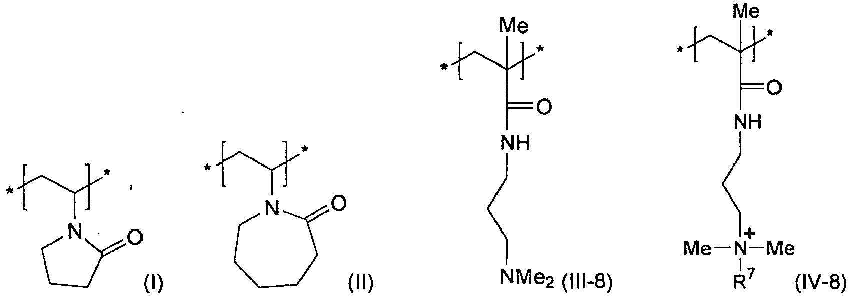 where R⁷ represents a (C₈ to C₃₀) alkyl group.

6. The agent according to one of claims 1 to 5, **characterized in that** it contains the amphiphilic, cationic polymers (a) in an amount of from 0.05% by weight to 15.0% by weight, particularly preferably from 0.05% by weight to 10.0% by weight, very particularly preferably from 0.1 to 5.0% by weight, based on the weight of the agent in each case.

7. The agent according to one of claims 1 to 6, **characterized in that** it contains the amphiphilic, anionic polymers (b) in an amount of from 0.05% by weight to 10.0% by weight, particularly preferably from 0.05% by weight to 5.0% by weight, very particularly preferably from 0.1 to 2.0% by weight, based on the weight of the agent in each case.

8. The agent according to one of claims 1 to 7, **characterized in that** the amphiphilic, cationic polymers (a) and the amphiphilic, anionic polymers (b) are present in a weight ratio of from 1 to 10 to 10 to 1, in particular of from 1 to 5 to 5 to 1, very particularly preferably of from 1 to 3.5 to 3.5 to 1.

9. The agent according to one of claims 1 to 8, **characterized in that** the amphiphilic, anionic polymers (b) are selected from the group of copolymers b1 having at least one structural unit of formula (V-1), at least one structural unit of formula (V-2), and at least one structural unit of formula (VI), 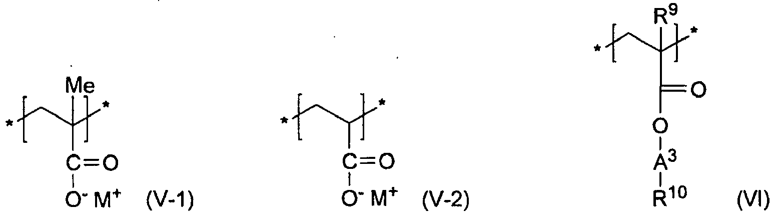 where
M⁺ independently represents a physiologically acceptable cation,
R⁹ represents a hydrogen atom or a methyl group,
R¹⁰ represents a (C₈ to C₃₀) alkyl group,
A³ represents a *-(CH₂CH₂O)ₓ-* group, where x represents an integer from 5 to 35, a *-(CH₂CHMeO)_{y}-* group, where y represents an integer from 5 to 35, or a *-(CH₂CH₂O)ₓ-(CH₂CHMeO)_{y}-* group, where the sum of x + y represents an integer from 5 to 35, and x and y are greater than zero.

10. The agent according to one of claims 1 to 9, **characterized in that** the amphiphilic, anionic polymers (b) are selected from formula (b1-1), 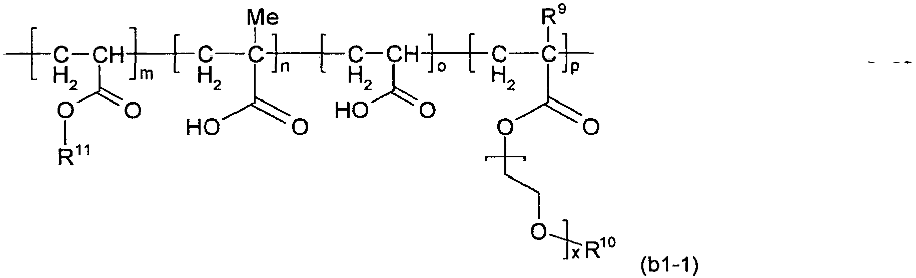 where
the indices m, n, o and p are each an integer greater than zero,
R⁹ represents a hydrogen atom or a methyl group,
R¹⁰ represents a hydrocarbon functional group having 8 to 30 hydrocarbon atoms,
R¹¹ represents a (C₁ to C₄) alkyl group, and
x represents 5 to 35.

11. The agent according to one of claims 1 to 10, **characterized in that** at least one film-forming and/or setting polymer is additionally contained therein.

12. The agent according to one of claims 1 to 11, **characterized in that** at least one surfactant, in particular at least one non-ionic surfactant, is additionally contained therein.

13. The agent according to one of claims 1 to 12, **characterized in that** it additionally contains at least one alkanolamine.

14. A method for treating keratin-containing fibers, in particular human hair, in which an agent according to at least one of claims 1 to 13 is applied to the keratin-containing fibers.

## Revendications

1. Agent de traitement de fibres de kératine, en particulier de cheveux humains, contenant dans un support cosmétiquement acceptable,
(a) au moins un polymère cationique amphiphile comportant au moins une unité structurelle de la formule (I), au moins une unité structurelle de la formule (II), au moins une unité structurelle de la formule (III) et au moins une unité structurelle de la formule (IV), 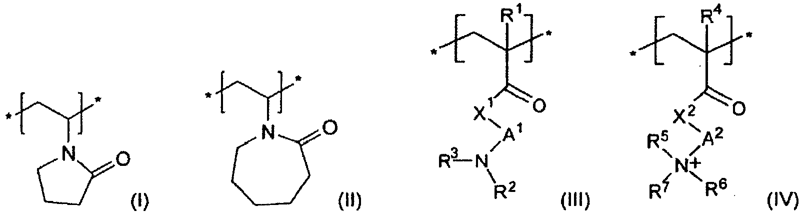 dans lesquelles
R¹ et R⁴ sont indépendamment un atome d'hydrogène ou un groupe méthyle,
X¹ et X² sont indépendamment un atome d'oxygène ou un groupe NH,
A¹ et A² sont indépendamment un groupe éthane-1,2-diyle, propane-1,3-diyle ou butane-1,4-diyle,
R², R³, R⁵ et R⁶ sont indépendamment un alkyle en C₁ à C₄,
R⁷ est un alkyle en C₈ à C₃₀
et
(b) au moins un polymère anionique amphiphile réticulé comportant au moins une unité structurelle de la formule (V) et au moins une unité structurelle de la formule (VI), 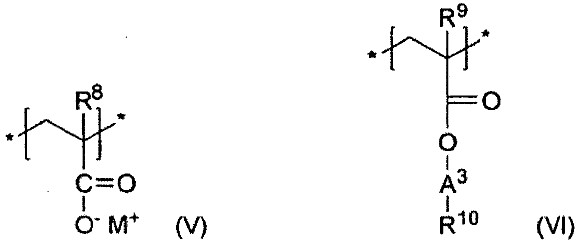 dans lesquelles
R⁸ et R⁹ sont indépendamment un atome d'hydrogène ou un groupe méthyle,
R¹⁰ est un groupement alkyle en C₈ à C₃₀,
M⁺ est un équivalent d'un cation physiologiquement acceptable et
A³ est un groupe *-(CH₂CH₂O)ₓ-*, dans lequel x est un nombre entier de 5 à 35, un groupe *-(CH₂CHMeO)_{y}-* dans lequel y est un nombre entier de 5 à 35 ou un groupe *-(CH₂CH₂O)ₓ-(CH₂CHMeO)_{y}-* dans lequel la somme x + y est un nombre entier de 5 à 35, et x et y sont supérieurs à zéro.

2. Agent selon la revendication 1, **caractérisé en ce que** A¹ et A² sont indépendamment l'éthane-1,2-diyle ou le propane-1,3-diyle.

3. Agent selon l'une des revendications 1 ou 2, **caractérisé en ce que** R², R³, R⁵ et R⁶ sont indépendamment un groupe méthyle ou éthyle, en particulier méthyle.

4. Agent selon l'une des revendications 1 à 3, **caractérisé en ce que** R⁷ est un décyle, un undécyle, un dodécyle, un tridécyle, un tétradécyle, un hexadécyle, un octadécyle, un eicosyle ou un docosyle.

5. Agent selon l'une des revendications 1 à 4, **caractérisé en ce que** le polymère cationique amphiphile est choisi parmi au moins un polymère comprenant au moins une unité structurelle de la formule (I), au moins une unité structurelle de la formule (II), au moins une unité structurelle de la formule (III-8) et au moins une unité structurelle de la formule (IV-8), 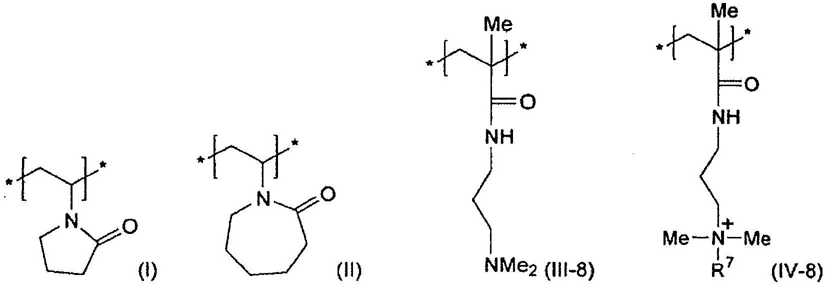 dans lesquelles R⁷ est un groupe alkyle en C₈ à C₃₀.

6. Agent selon l'une des revendications 1 à 5, **caractérisé en ce qu'**il contient les polymères cationiques (a) amphiphiles dans une quantité de 0,05 à 15,0% en poids, de façon particulièrement préférée de 0,05% à 10,0% en poids, de façon tout particulièrement préférée de 0,1% à 5,0%, par rapport au poids de l'agent.

7. Agent selon l'une des revendications 1 à 6, **caractérisé en ce qu'**il contient les polymères anioniques amphiphiles (b) dans une quantité de 0,05% à 10,0% en poids, de façon particulièrement préférée de 0,05% à 5,0% en poids, de façon tout particulièrement préférée de 0,1% à 2,0% en poids, par rapport au poids de l'agent.

8. Agent selon l'une des revendications 1 à 7, **caractérisé en ce que** les polymères cationiques amphiphiles (a) et les polymères anioniques amphiphiles (b) sont utilisés dans un rapport en poids de 1:10 à 10:1, en particulier de 1:5 à 5:1, de façon tout particulièrement préférée de 1:3,5 à 3,5:1.

9. Agent selon l'une des revendications 1 à 8, **caractérisé en ce que** les polymères anioniques amphiphiles (b) sont choisis dans le groupe des copolymères b1, qui comportent au moins une unité structurelle de la formule (V-1), au moins une unité structurelle de la formule (V-2) et au moins une unité structurelle de la formule (VI) 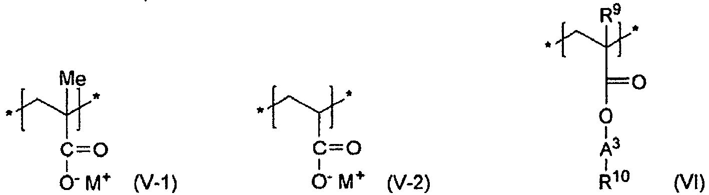 dans lesquelles
M⁺ est indépendamment un cation physiologiquement acceptable,
R⁹ est un atome d'hydrogène ou un groupe méthyle,
R¹⁰ est un groupe alkyle en C₈ à C₃₀,
A³ est un groupe *-(CH₂CH₂O)ₓ-* dans lequel x est un nombre entier de 5 à 35, un groupe *-(CH₂CHMeO)_{y}-* dans lequel y est un nombre entier de 5 à 35 ou un groupe *-(CH₂CH₂O)ₓ-(CH₂CHMeO)_{y}-* dans lequel la somme x + y est un nombre entier de 5 à 35, et x et y sont supérieurs à zéro.

10. Agent selon l'une des revendications 1 à 9, **caractérisé en ce que** les polymères amphiphiles anioniques (b) sont choisis à partir de la formule (b1-1), 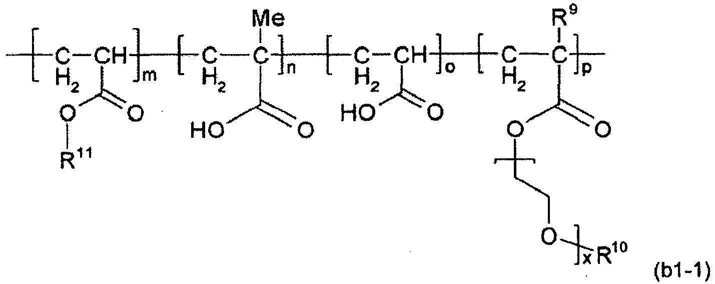
dans laquelle m, n, o et p sont chacun un nombre entier supérieur à zéro,
R⁹ est un atome d'hydrogène ou un groupe méthyle,
R¹⁰ est un radical hydrocarboné ayant de 8 à 30 atomes de carbone,
R¹¹ est un groupe alkyle en C₁ à C₄,
x est un nombre de 5 à 35.

11. Agent selon l'une des revendications 1 à 10, **caractérisé en ce qu'**il contient en plus l'au moins un polymère de formation de film et/ou de renforcement.

12. Agent selon l'une des revendications 1 à 11, **caractérisé en ce qu'**il contient en plus au moins un tensioactif, en particulier au moins un tensioactif non ionique.

13. Agent selon l'une des revendications 1 à 12, **caractérisé en ce qu'**il contient en plus au moins une alcanolamine.

14. Procédé de traitement de fibres de kératine, en particulier de cheveux humains, dans lequel un agent selon au moins une des revendications 1 à 13 est appliqué sur les fibres de kératine.
